# EUROPÄISCHE PATENTSCHRIFT

(11) **EP 1 499 254 B1**
(45) Veröffentlichungstag und Bekanntmachung des Hinweises auf die Patenterteilung: **04.06.2008**
(21) Anmeldenummer: 03747081.2
(22) Anmeldetag: 28.04.2003
(51) Int. Cl.: A61B 18/12

(54) **HOCHFREQUENZ-CHIRURGIEGENERATOR**
HIGH-FREQUENCY SURGICAL GENERATOR
GENERATEUR HAUTE FREQUENCE POUR LA CHIRURGIE HAUTE FREQUENCE

(30) Priorität: 26.04.2002 DE 10218895
(43) Veröffentlichungstag der Anmeldung: 26.01.2005
(73) Patentinhaber: Storz Endoskop Produktions GmbH, 78532 Tuttlingen (DE)
(72) Erfinder: DANERS, Felix, CH-8200 Schaffhausen (CH); NOVAK, Pavel, CH-8234 Stetten (CH)
(74) Vertreter: Lohr, Georg
(86) Internationale Anmeldenummer: PCT/DE2003/001367
(87) Internationale Veröffentlichungsnummer: WO 2003/090635

(56) Entgegenhaltungen:
- EP-A- 1 082 944
- US-A- 4 727 874
- US-A- 6 090 106
- US-A- 6 093 186

## Beschreibung

### Technisches Gebiet

Die Erfindung betrifft einen Generator zur Leistungserzeugung für die Hochfrequenzchirurgie. In der Hochfrequenzchirurgie wird menschliches oder tierisches Körpergewebe mittels elektrischem Strom geschnitten bzw. koaguliert. Die Hochfrequenzchirurgie ist insbesondere in Verbindung mit endoskopischen Operationstechniken äußerst vorteilhaft einsetzbar.

### Stand der Technik

Die Aufgabe der Hochfrequenzchirurgiegeneratoren ist es, die elektrische Energie für die Hochfrequenzchirurgie derart bereitzustellen, dass das gewünschte Operationsergebnis erreicht wird. Um Muskel- bzw. Nervenreizungen zu minimieren, liefern Hochfrequenzchirurgiegeneratoren hochfrequente Energie im Frequenzbereich über 300 kHz. Diese hochfrequente Energie wird meist mittels einer Elektrode in das Gewebe eingespeist. Am Punkt der Einspeisung tritt eine starke Erwärmung des die Elektrode umgebenden Gewebes auf. Wird in einem kurzen Zeitintervall eine hohe Energie zugeführt, so führt dies zu einem Verdampfen der Zellflüssigkeit und einem Aufplatzen der Zellen, so dass sich der Zellverband um die Elektrode auflöst. Die Elektrode kann sich nahezu frei durch das Gewebe bewegen. Wird über längere Zeit eine geringere Energie zugeführt, so führt dies zu einer Koagulation des Gewebes d. h. zu einer Gerinnung des Eiweißes. Die Zellen sterben hierbei ab und werden zu einer zähen Masse.

Grundsätzlich werden bezüglich der Einleitung der hochfrequenten Energie zwei Anordnungen unterschieden.

Bei der monopolaren Anordnung wird eine kleinflächige Schneide- bzw. Koagulationselektrode zur Stromeinleitung an Operationsort und eine großflächige "neutrale Elektrode" zur Stromausleitung an einem anderen Ort des Körpers des Patienten angeordnet. Die Elektrodenfläche ist hier so groß dimensioniert, dass es zu keiner nennenswerten Wärmeentwicklung an der Elektrode kommt.

Die bipolare Anordnung umfasst eine zweigeteilte Elektrode, bei der die Stromeinleitung sowie die Ausleitung am Operationsort erfolgt.

Der Dosierung der Energie kommt größte Bedeutung zu, da diese das Operationsergebnis unmittelbar beeinflusst. Gibt der Generator zu wenig Energie ab, so ist kein Schneiden möglich, wird zu viel Energie abgegeben, so werden die Schnittränder stark koaguliert, was wiederum zu einer erschwerten Abheilung bzw. erhöhtem Infektionsrisiko führt.

Somit ist es das Ziel, für einen reinen Schneidvorgang so wenig Energie wie möglich und für einen kombinierten Schneide- bzw. Koagulationsvorgang die minimale, zur Koagulation benötigte Energie in das Gewebe einzubringen.

Um diese Energie zu minimieren, wird in der US-Patentschrift 4,114,623 ein Verfahren zur Regelung des Generatorstromes durch Beobachtungen des beim Schneiden auftretenden Lichtbogens offenbart.

Ein besonderes Problem stellt hier der Schnittbeginn bzw. der Übergang in eine andere Gewebeart mit anderen elektrischen Eigenschaften dar. Da beim Übergang in eine andere Gewebeart nahezu die gleiche Aufgabenstellung wie beim Anschneiden vorliegt, wird nachfolgend nur noch auf das Anschneiden Bezug genommen.

Wird mit zu hoher Leistung angeschnitten, so ergibt sich an der Anschnittstelle bereits eine unerwünschte Koagulation. Um diese Koagulation zu minimieren, wird in der DE 38 15 835 A1 eine Begrenzung der Generatorausgangsspannung vorgeschlagen. Diese verhindert ein Anschneiden mit zu hoher Generatorleistung. Wird stattdessen mit zu niedriger Leistung angeschnitten, so führt dieses zu keinem Schneidevorgang durch Eindringen der Elektrode in das Gewebe, sondern vielmehr zu einer unerwünschten Koagulation der Gewebeoberfläche. Diese erschwert auch ein weiteres Anschneiden. Um ein sicheres, gewebeunabhängiges Anschneiden zu gewährleisten, wird in der DE 41 35 184 A1 vorgeschlagen, zu Beginn des Anschneidens eine erhöhte Generatorleistung abzugeben. Diese erhöhte Leistungsabgabe kann dann bei Erkennung eines Lichtbogens auf den normal zum Schneiden benötigten Wert abgesenkt werden.

In der Realisierung lässt sich hier allerdings eine Koagulation an der Anschnittstelle kaum vermeiden, da die Absenkung der Leistungsabgabe herkömmlicher Generatoren nicht schnell genug erfolgen kann. Dies soll beispielhaft an einem konventionellen Chirurgiegenerator bestehend aus einer Gleichspannungsversorgung mit nachgeschaltetem Leistungsoszillator dargestellt werden. Hier muss zu Absenkung der Generatorausgangsspannung zunächst die Ausgangsspannung des Gleichspannungsnetzteils abgesenkt werden. Dazu müssen die Filterkondensatoren entladen werden. Weiterhin muss aus den Blindelementen des Filterkreises im Leistungsoszillator die Energie entnommen werden.

Zur Entladung der Filterkondensatoren.in Netzteilen sind gesteuerte Lastwiderstände bekannt. So kann beispielsweise ein Lastwiderstand in Serie mit einem Leistungstransistor parallel zum Ausgang des Netzteils geschaltet sein. Zu Spannungsabsenkung wird der Leistungstransistor angesteuert und entlädt die Filterkondensatoren des Netzteils. Bei den bisher bekannten Generatorschaltungen ist die Zeitkonstante des Netzteils wesentlich höher als die Zeitkonstante zur Entladung der Blindelemente im Filterkreis des Leistungsoszillators, so dass der Optimierung dieser Zeitkonstante bisher wenig Beachtung geschenkt wurde.

Die bisher bekannten Lösungen weisen relativ hohe Zeitkonstanten auf, so dass eine Koagulation am Schnittbeginn bzw. bei Gewebeänderungen kaum vermeidbar ist. Weiterhin weisen Sie eine hohe Verlustleistung durch die thermischen Verluste in Leistungstransistor und Widerstand zur Entladung der Filterkondensatoren des Netzteils auf.

Um eine Koagulation am Schnittbeginn bzw. bei Gewebeänderungen zu vermeiden, ist eine schnelle Regelung der Ausgangsspannung unbedingt notwendig. Weiterhin sollen moderne Hochfrequenzchirurgiegeneratoren immer kleiner und preisgünstiger werden. Durch eine Realisierung mit hohem Wirkungsgrad der Regelung der Ausgangsspannung kann auf zusätzliche Kühlkörper verzichtet werden.

### Darstellung der Erfindung

Der Erfindung liegt die Aufgabe zugrunde, einen Hochfrequenzchirurgiegenerator bereitzustellen, welcher eine schnelle Regelung der Ausgangsspannung bei hohem Wirkungsgrad ermöglicht.

Eine erfindungsgemäße Lösung dieser Aufgabe ist im Patentanspruch 1 angegeben. Weiterbildungen der Erfindung sind Gegenstand der abhängigen Ansprüche.

Die erfindungsgemäße Vorrichtung umfasst einen Hochfrequenzgenerator (1) für die Hochfrequenzchirurgie. Dieser umfasst weiterhin zumindest einen Leistungsoszillator (2), zur Abgabe hochfrequenter Energie, welcher von einer Gleichspannungsversorgung (3) versorgt wird. Diese Spannungsversorgung wandelt eine erste Eingangspannung (4) in die zur Versorgung des Leistungsoszillators benötigte Spannung (5) um. Die entsprechenden Leistungsoszillatoren weisen meist einen sehr hohen Wirkungsgrad sowie einen niedrigen Innenwiderstand auf, so dass ihre Ausgangsspannung proportional der Eingangspannung ist. Zur Steuerung der Ausgangsspannung des Hochfrequenzgenerators ist die Gleichspannungsversorgung ebenfalls steuerbar. So kann diese nach Bedarf entsprechend der Vorgabe durch eine Steuereinheit unterschiedliche Ausgangsspannungen abgeben.

Eine erfindungsgemäße Gleichspannungsversorgung weist nun mindestens zwei Betriebsarten auf. Eine erste Betriebsart dient in herkömmlicher Weise zum Transfer der benötigten Energie von der ersten Eingangspannung zur Versorgung des Leistungsoszillators. Um nun schnelle Änderungen der Ausgangsspannung zur Anpassung an sich schnell ändernde Operationssituationen zu ermöglichen, ist eine weitere Betriebsart vorgesehen. Diese transferiert Energie von der Versorgung des Leistungsoszillators zurück zur ersten Eingangspannung. In dieser Betriebsart können die Energiespeicher auf der Ausgangseite der Gleichspannungsversorgung sowie im Leistungsoszillator schnell entladen werden. Dadurch lässt sich die Ausgangsspannung der Gleichspannungsversorgung und somit auch die Ausgangsspannung des Leistungsoszillators in kürzester Zeit absenken.

Eine weitere vorteilhafte Ausgestaltung der Erfindung besteht darin, dass auch der Leistungsoszillator (2) wenigstens zwei Betriebsarten aufweist. Dabei dient eine Betriebsart in konventioneller Weise zum Transfer von Energie an den Ausgang (6), während eine andere Betriebsart zur Rückspeisung der in den Blindelementen gespeicherten Energie in die Gleichspannungsversorgung vorgesehen ist. Dadurch kann auch die Ausgangsspannung des Leistungsoszillators bei hohen Lastimpedanzen schnell auf niedrige Werte abgesenkt werden. Ist eine solche zweite Betriebsart nicht vorgesehen, so kann die Abgabe der Energie aus den Blindelementen ausschließlich an die Last d.h. an den Patienten erfolgen. Dies führt dann zu unerwünschten Koagulationen bzw. Verbrennungen. Aus diesen Gründen wurde bisher versucht, die in den Blindelementen gespeicherte Energie so klein wie möglich zu halten. Durch die Möglichkeit der Rückspeisung der Energie besteht nun ein neuer Freiheitsgrad zur Dimensionierung und Optimierung der Filter bzw. Blindelemente im Ausgangskreis des Leistungsoszillators. Eine solche zweite Betriebsart kann beispielsweise durch zusätzliche Hilfsschalter (Leistungstransistoren) zur Entladung der Blindelemente realisiert werden. Ebenso ist eine solche Entladung durch eine entsprechende gegenphasige Ansteuerung der Leistungsendstufe realisierbar. Voraussetzung für eine solche Rückspeisung ist, dass die in die Gleichspannungsversorgung rückgespeiste Energie in groß dimensionierten Energiespeichern, wie beispielsweise Kondensatoren, aufgefangen bzw. weiter an deren Eingang rückgespeist werden kann.

Eine andere vorteilhafte Ausgestaltung der Erfindung besteht darin, dass die Gleichspannungsversorgung (3) als modifizierter Buck-Wandler ausgelegt ist, wobei die Freilaufdiode durch einen Schalter ersetzt ist. In dieser Konfiguration ergibt sich einerseits ein verbesserter Wirkungsgrad, da moderne Schalter wie beispielsweise MOSFETs geringere Verluste als Dioden aufweisen und andererseits die Möglichkeit des inversen Betriebs zur Rückspeisung von Energie.

Eine weitere vorteilhafte Ausgestaltung der Erfindung besteht darin, dass ein zusätzlicher Spannungswandler (7) als Leistungsfaktorkorrekturschaltung vorgesehen ist. Dieser wandelt eine Sinusförmige Netzspannung (8) mit hohem Leistungsfaktor (cos(Phi)≈1) in eine gleichgerichtete erste Eingangspannung (4) um.

In einer vorteilhaften Ausgestaltung der Erfindung sind der zusätzliche Spannungswandler (7) als Leistungsfaktorkorrekturschaltung und die Gleichspannungsversorgung (3) mit ihren Takten synchronisiert. Durch diese Synchronisation kann eine relativ hohe Welligkeit der gleichgerichteten ersten Eingangspannung (4) toleriert werden. Diese führt zu keinen Instabilitäten der Gleichspannungsversorgung, da die über eine Periode des Taktes gemittelte Eingangspannung immer konstant ist. Weiterhin wird durch die Synchronisation der Ripple-Strom in den Kondensatoren erheblich reduziert. Somit können die Energiespeichernden Elemente, insbesondere die Filterkondensatoren kleiner und preisgünstiger dimensioniert werden. Ferner erhöht sich auf Grund der geringeren Strombelastung die Lebensdauer der Kondensatoren.

Eine weitere Ausgestaltung der Erfindung umfasst eine Gleichspannungsversorgung (3), welche durch einen Zustandsregler (9) in mindestens einer ihrer Ausgangsgrößen geregelt wird. Derartige Ausgangsgrößen sind beispielsweise Ausgangsspannung, Ausgangsstrom oder auch die Ausgangsleistung. Ein solcher Zustandsregler benutzt zur Regelung nicht nur Istwerte der zu regelnden Ausgangsgröße, sondern auch mindestens einen zusätzlichen Istwert einer Spannung oder eines Stromes einer internen Schaltungskomponente. So wird vorzugsweise im Falle des Einsatzes eines Buck-Wandlers der Strom durch die Induktivität mit erfasst. Durch derartige zusätzliche Istwerte lassen sich bessere Regeleigenschaften, wie höhere Stabilität, bessere Ausregelung und höhere Regelgeschwindigkeit erreichen.

In einer weiteren Ausgestaltung der Erfindung ist zur Funktionskontrolle der Gleichspannungsversorgung (3) eine zusätzliche steuerbare Stromsenke bzw. Last (10) am Ausgang der Gleichspannungsversorgung angeordnet. Mittels einer solchen Last kann ohne Aktivierung des Leistungsoszillators und somit ohne Leistungsabgabe an die Ausgangsklemmen bzw. den Patientenstromkreis die Funktion der Gleichspannungsversorgung sowie der Spannungs- und Strommesseinrichtungen überprüft werden. Zur Überprüfung wird die Stromsenke bzw. Last von einer Steuereinheit aktiviert bzw. ein vorgegebener Lastwiderstand eingestellt. Bei aktivierter Gleichspannungsversorgung kann durch Auswertung der Spannungsmessung bzw. Strommessung deren Funktion überprüft werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.

Fig. 1 zeigt in allgemeiner Form schematisch eine erfindungsgemäße Vorrichtung.

Fig. 2 zeigt beispielhaft eine besonders vorteilhafte Gleichspannungsversorgung.

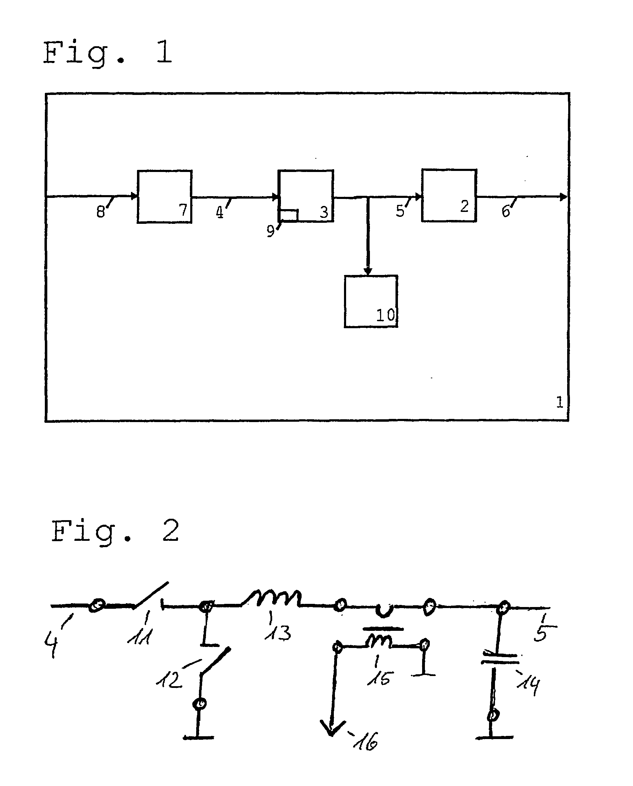

In der Fig. 1 ist eine erfindungsgemäße Vorrichtung beispielhaft abgebildet. Ein Generator für die Hochfrequenzchirurgie (1) (Hochfrequenzchirurgiegenerator) umfasst eine Gleichspannungsversorgung (3), welche eine erste Eingangspannung (4) in eine Spannung (5) zur Versorgung des Leistungsoszillators umsetzt. Der Leistungsoszillator (2) erzeugt daraus ein hochfrequentes Signal, welches an seinem Ausgang (6) abgegeben wird. Die Gleichspannungsversorgung weist hier beispielhaft einen Zustandsregler auf. Weiterhin ist am Ausgang der Gleichspannungsversorgung eine steuerbare Stromsenke bzw. Last zur Überprüfung der Funktion angeordnet. Um eine Sinusförmige Stromaufnahme der Sinusförmigen Netzspannung (8) zu erreichen, ist vor die Gleichspannungsversorgung ein zusätzlicher Spannungswandler als Leistungsfaktorkorrekturschaltung (7) angeordnet.

Fig. 2 zeigt beispielhaft einen besonders geeigneten Buck-Wandler. Hierin wird eine erste Eingangspannung (4) mit einem ersten Leistungsschalter (11) zerhackt und durch die Serieninduktivität (13) sowie die Parallelkapazität (14) gefiltert und als Spannung zur Versorgung des Leistungsgenerators (5) abgegeben. Die üblicherweise bei Buck-Wandlern vorgesehene Freilaufdiode wird hier durch einen zweiten Leistungsschalter (12) an gleicher Stelle ersetzt. Alternativ hierzu könnte auch der zweite Leistungsschalter (12) parallel zu der üblicherweise vorhandenen Freilaufdiode geschaltet sein.
In der ersten Betriebsart zum Energietransfer von der ersten Eingangspannung (4) hin zur Versorgung des Leistungsgenerators (5) kann der zweite Leistungsschalter (12) auch geöffnet bleiben, falls ihm eine Diode in der bei diesen Wandlern üblichen Weise parallel geschaltet ist. In einer ersten Schaltphase ist der erste Leistungsschalter (11) geschlossen - der Strom fließt weiter durch die Serieninduktivität (13) in die Parallelkapazität (14). Wird in einer zweiten Schaltphase nun dieser erste Leistungsschalter geöffnet, so versucht die Induktivität den Stromfluss aufrechtzuerhalten. Die Spannung am Schalterseitigen Ende der Induktivität kommutiert um und wird so negativ, dass eine anstelle des zweiten Leistungsschalters (12) bzw. parallel hierzu angeordnete Diode leitend wird. Wird nun ein zweiter Leistungsschalter (12) parallel zur Diode eingeschaltet, so lassen sich die Verluste der Anordnung wesentlich reduzieren, da moderne Leistungsschalter, wie beispielsweise MOSFETs wesentlich niedrigere Verluste als Dioden aufweisen. MOSFETs weisen regelmäßig durch ihren internen Aufbau parasitäre parallele Dioden auf, welche die oben beschriebenen Funktionen übernehmen können.
In der zweiten Betriebsart zum Energietransfer von der Versorgung des Leistungsoszillators (5) zurück zur ersten Eingangspannung (4) kann die Gleichspannungsversorgung nun in umgekehrter Richtung wie ein Boost-Wandler betrieben werden. Bei derartigen Wandlern ist anstelle des ersten Leistungsschalters (11) eine Diode vorgesehen. Die Wirkungsweise ist analog zu der zuvor beschriebenen und entspricht der Betriebsart bekannter Boost-Wandler.
Weiterhin ist in Serie zur Serieninduktivität (13) ein Strommesswandler (15) vorgesehen, welcher ein Strommesssignal (16) an den Zustandsregler abgibt.

### Bezugszeichenliste

- 1: Hochfrequenzgenerator
- 2: Leistungsoszillator
- 3: Gleichspannungsversorgung
- 4: erste Eingangspannung
- 5: Spannung zur Versorgung des Leistungsoszillators
- 6: Ausgang des Leistungsoszillators
- 7: zusätzlicher Spannungswandler als Leistungsfaktorkorrekturschaltung
- 8: Sinusförmige Netzspannung
- 9: Zustandsregler
- 10: Stromsenke bzw. Last
- 11: erster Leistungsschalter
- 12: zweiter Leistungsschalter
- 13: Serieninduktivität
- 14: Parallelkapazität
- 15: Strommesswandler
- 16: Strommesssignal

## Patentansprüche

1. Hochfrequenzgenerator (1) für die Hochfrequenzchirurgie umfassend einen Leistungsoszillator (2) zur Abgabe hochfrequenter Energie gespeist von einer Gleichspannungsversorgung (3), welche eine erste Eingangsspannung (4) in die zur Versorgung des Leistungsoszillators benötigte Spannung (5) umsetzt,
**dadurch gekennzeichnet, dass**
die Gleichspannungsversorgung (3) mindestens zwei Betriebsarten aufweist, wobei eine erste Betriebsart für den Energietransfer von der ersten Eingangsspannung (4) hin zur Versorgung des Leistungsoszillators (5) und eine zweite Betriebsart für den Energietransfer von der Versorgung des Leistungsoszillators (5) zurück zur ersten Eingangspannung (4) vorgesehen ist.

2. Hochfrequenzgenerator nach Anspruch 1,
**dadurch gekennzeichnet, dass**
der Leistungsoszillator (2) mindestens eine erste Betriebsart zu Abgabe hochfrequenter Energie an den Ausgang (6) und eine zweite Betriebsart zur Rückspeisung der in den Blindelementen gespeicherten Energie in die Gleichspannungsversorgung (3) aufweist.

3. Hochfrequenzgenerator nach Anspruch 1 bzw. 2,
**dadurch gekennzeichnet, dass**
die Gleichspannungsversorgung (3) als modifizierter Buck-Wandler, bei dem die Freilaufdiode durch einen Schalter (12) ersetzt ist, ausgebildet ist.

4. Hochfrequenzgenerator nach einem der Ansprüche 1 bis 3,
**dadurch gekennzeichnet, dass**
ein zusätzlicher Spannungswandler (7) als Leistungsfaktorkorrekturschaltung vorgesehen ist, welcher eine sinusförmige Netzspannung (8) in eine gleichgerichtete erste Eingangsspannung (4) umsetzt.

5. Hochfrequenzgenerator nach Anspruch 4,
**dadurch gekennzeichnet, dass**
der zusätzliche Spannungswandler (7) als Leistungsfaktorkorrekturschaltung und die Gleichspannungsversorgung (3) mit ihren Takten synchronisiert sind.

6. Hochfrequenzgenerator nach einem der Ansprüche 1 bis 5,
**dadurch gekennzeichnet, dass**
zur Regelung der Gleichspannungsversorgung ein Zustandregler (9) vorgesehen ist, welcher als Messgrößen zur Regelung nicht nur die Ausgangsspannung bzw. den Ausgangsstrom der Gleichspannungsversorgung (3), sondern auch Spannungs- bzw. Stromwerte von internen Komponenten heranzieht.

7. Hochfrequenzgenerator nach einem der Ansprüche 1 bis 6,
**dadurch gekennzeichnet, dass**
eine zusätzliche steuerbare Stromsenke bzw. Last (10) zur Funktionskontrolle am Ausgang der Gleichspannungsversorgung (3) angeordnet ist.

## Claims

1. A high-frequency generator (1) for high-frequency surgery, comprising a power oscillator (2) for emitting high-frequency energy which is supplied by a direct voltage supply (3) which converts a first input voltage (4) into the voltage (5) required for supplying the power oscillator, **characterized in that** the direct voltage supply (3) has at least two operating modes, with a first operating mode being provided for energy transfer from the first input voltage (4) towards the supply of the power oscillator (5) and a second operating mode for the energy transfer from the supply of the power oscillator (5) back to the first input voltage (4).

2. A high-frequency generator according to claim 1, **characterized in that** the power oscillator (2) has at least a first operating mode for emitting high-frequency energy to the output (6) and a second operating mode for reclamation of the energy stored in the reactive components to the direct voltage supply (3).

3. A high-frequency generator according to claim 1 or 2, **characterized in that** the direct voltage supply (3) is arranged as a modified Buck converter in which the freewheeling diode is replaced by a switch (12).

4. A high-frequency generator according to one of the claims 1 to 3, **characterized in that** an additional voltage transformer (7) is provided as a power-factor correction circuit which converts a sinusoid mains voltage (8) into a rectified first input voltage (4).

5. A high-frequency generator according to claim 4, **characterized in that** the additional voltage converter (7) as a power-factor correction circuit and the direct voltage supply (3) are synchronized with their cycles.

6. A high-frequency generator according to one of the claims 1 to 5, **characterized in that** a state regulator (9) is provided for regulating the direct voltage supply, which regulator uses as measured quantities for regulating not only the output voltage or the output current of the direct voltage supply (3) but also the voltage or current values of internal components.

7. A high-frequency generator according to one of the claims 1 to 6, **characterized in that** an additional controllable current sink or load (10) for checking the function is arranged at the output of the direct voltage supply (3).

## Revendications

1. Générateur de hautes fréquences (1) pour la chirurgie à haute fréquence, comprenant un générateur de puissance (2) destiné à fournir une énergie à haute fréquence alimenté par une alimentation en courant continu (3) qui transforme une première tension d'entrée (4) en tension (5) nécessaire pour l'alimentation de l'oscillateur de puissance, **caractérisé en ce que** l'alimentation en courant continu (3) présente au moins deux modes de fonctionnement, le premier mode de fonctionnement étant prévu pour le transfert d'énergie à partir de la première tension d'entrée (4) pour alimenter l'oscillateur de puissance (5) et un deuxième mode de fonctionnement pour le transfert d'énergie en retour de l'alimentation de l'oscillateur de puissance (5) à la première tension d'entrée (4).

2. Générateur de hautes fréquences selon la revendication 1, **caractérisé en ce que** l'oscillateur de puissance (2) présente au moins un premier mode de fonctionnement pour la délivrance d'énergie à haute fréquence à la sortie (6) et un deuxième mode de fonctionnement pour le renvoi de l'énergie stockée dans les éléments réactifs vers l'alimentation en courant continu (3).

3. Générateur de hautes fréquences selon la revendication 1 ou 2, **caractérisé en ce que** l'alimentation en courant continu (3) est conçue comme un convertisseur Buck modifié, dans lequel la diode de roue libre est remplacée par un commutateur (12).

4. Générateur de hautes fréquences selon l'une des revendications 1 à 3, **caractérisé en ce qu'**il est prévu un convertisseur de tension supplémentaire (7) servant de circuit de correction du facteur de puissance, qui convertit une tension de réseau sinusoïdale (8) en première tension d'entrée (4) redressée.

5. Générateur de hautes fréquences selon la revendication 4, **caractérisé en ce que** les cadences du convertisseur de tension supplémentaire (7) servant de circuit de correction du facteur de puissance et de l'alimentation en courant continu (3) sont synchronisées.

6. Générateur de hautes fréquences selon l'une des revendications 1 à 5, **caractérisé en ce qu'**il est prévu pour réguler l'alimentation en courant continu un régulateur d'état (9) qui utilise comme grandeurs de mesure pour la régulation, non seulement la tension de sortie ou l'intensité de sortie de l'alimentation en courant continu (3), mais aussi les valeurs de tension ou d'intensité de composants internes.

7. Générateur de hautes fréquences selon l'une des revendications 1 à 6, **caractérisé en ce qu'**un puits de courant ou une charge (10) réglable supplémentaire est disposé pour le contrôle du fonctionnement à la sortie de l'alimentation en courant continu (3).
